# EUROPEAN PATENT SPECIFICATION

(11) **EP 1 547 614 B1**
(45) Date of publication and mention of the grant of the patent: **19.01.2011**
(21) Application number: 03733109.7
(22) Date of filing: 28.05.2003
(51) Int. Cl.: A61K 31/198, A61P 1/16, A61P 3/00, A61P 3/06, A61P 3/10, A61P 5/48, A61P 43/00

(54) **MEDICINAL COMPOSITION FOR INHIBITING THE EXPRESSION OF ATP-CITRATE LYASE AND USE THEREOF**
MEDIZINISCHE ZUSAMMENSETZUNGEN ZUR HEMMUNG DER EXPRESSION VON ATP-CITRAT-LYASE UND IHRE VERWENDUNG
COMPOSITION MEDICINALE POUR INHIBER L'EXPRESSION D'ATP-CITRATE LYASE ET SON UTILISATION

(30) Priority: 28.05.2002 JP 2002154397
(43) Date of publication of application: 29.06.2005
(73) Proprietor: Ajinomoto Co., Inc., Tokyo 104-0031 (JP)
(72) Inventor: KITAHARA, Yoshiro, AJINOMOTO CO., INC., Kawasaki-shi, Kanagawa 210-0801 (JP); OKUTSU, Tomohisa, AJINOMOTO CO., INC., Kawasaki-shi, Kanagawa 210-0801 (JP); MITSUI, Akira, AJINOMOTO CO., INC., Kawasaki-shi, Kanagawa 210-0801 (JP); OKANO, Akira, AJINOMOTO CO., INC., Kawasaki-shi, Kanagawa 210-0801 (JP)
(74) Representative: Nicholls, Kathryn Margaret
(86) International application number: PCT/JP2003/006651
(87) International publication number: WO 2003/099332

(56) References cited:
- EP-A2- 0 196 222
- WO-A-01/21159
- WO-A-01/26639
- WO-A-93/22304
- WO-A1-01/34579
- WO-A1-01/68136
- WO-A1-99/58122
- SULLIVAN A C ET AL: "HYPOLIPIDEMIC ACTIVITY OF (---)-HYDROXYCITRATE" LIPIDS, CHAMPAIGN, IL, US, vol. 12, no. 1, 1977, pages 1-9, XP000613919 ISSN: 0024-4201
- AL-SHURBAJI AYMAN ET AL: "Effect of 3-thiadicarboxylic acid on lipid metabolism in experimental nephrosis" ARTERIOSCLEROSIS AND THROMBOSIS, vol. 13, no. 11, 1993, pages 1580-1586, XP009087126 ISSN: 1049-8834
- IKENOUE TAKAO ET AL.: 'Hypoglycemic and insulinotropic effects of a novel oral antidiabetic agent, (-)-N-(trans-4-isopropylcyclohexanecarbonyl )-D-phenylalanine (A-4166)' BRITISH JOURNAL OF PHARMACOLOGY vol. 120, no. 1, 1997, pages 137 - 145, XP000917761

## Description

### Background of the Invention

The present invention relates to a pharmaceutical composition for use in preventing, alleviating or treating NASH or another liver discorder.

As the dietary habits have been westernized recently, patients with metabolic syndrome having symptoms of a severe insulin resistance, obesity, hypertension, hyperlipemia and hyperglycemia and also patients with syndrome X, insulin resistant syndrome and multiple risk factor syndrome are increasing in number. In particular, patients having symptoms related to abnormal lipid metabolism such as obesity and fatty liver have a high risk of suffering from non-alcoholic steato-hepatitis (NASH). Under these circumstances, it is an important problem to develop a method for preventing, improving and treating these hepatic diseases (Gastroenterology, 121:710 (2001)).

As medicines for improving abnormal lipid metabolism, there have been known statin(s) which are inhibitors from HMGCoA, i. e. an enzyme relating to cholesterol synthesis, fibrates which activate transcription factor PPARα and glitazones which activate PPARγ. Although these medicines were reported to improve hypercholesterolemia, hypertriglyceridemia and hyperglycemia with insulin resistance, they do not improve the whole abnormal lipid metabolism accompanying metabolic syndrome. Further, although it was reported that some of the above-described medicines or metformin used as a hypoglycemic agent is also effective on NASH (Hepatology, 33: 1338 (2001)), the effect thereof is not yet satisfactory.

ATP citrate lyase (hereinafter referred to as "ACL") is positioned at the uppermost part of the stream of the fat synthesis pathway. ACL is an enzyme which forms acetyl CoA used as a starting material in the synthesis of lipids in the cells from citric acid which is an intermediate in the glycolytic pathway. As inhibitors for this enzyme, there have been reported compounds from microorganisms (A. Antibiot., 50: 729 (1997), Japanese Patent Kokai No. JP 2001-261682) and citric acid analogs (Eur. J. Biochem., 202: 889 (1991), J. Med. Chem., 35: 4875 (1992) and J. Med. Chem., 38, 537 (1995)). It was reported that in those inhibitors, gamma-lactone prodrugs of (3R,5S)-omega-substituted-3-carboxy-3,5-dihydroxyalkanoic acids inhibit the synthesis of cholesterol and fatty acids from liver cell line HepG2 and that blood cholesterol and triglyceride level of rats or dogs can be lowered by the *in vivo* administration of the prodrugs (J. Med. Chem., 41:3582 (1998)). A therapeutic agent for hyperlipemia which contains the prodrug and a method for treating hyperlipemia with this agent were applied for patent (WO 93/22304). However, compounds capable of controlling the ACL expression level *per se* by the in vivo administration thereof have not yet been known. The expression of ACL is physiologically elevated when carbohydrates are taken after the fasting. It is known that the expression is constitutively elevated in animals with metabolic syndrome (J. Biol. Chem., 274: 30028 (1999), J. Biol. Chem., 274:35832 (1999) and J. Biol. Chem., 276:38337 (2001)). However, it has not yet been reported to find a compound which suppresses the elevated ACL expression and to use this compound for the prevention, improvement and treatment of metabolic syndrome, in particular, obesity and liver diseases such as fatty liver and NASH.

On the other hand, nateglinide is a compound capable of reacting on pancreatic β cells to rapidly stimulate the insulin secretion (Br. J. Pharmacol., 120: 137 (1997)). The use of nateglinide alone as a hypoglycemic agent (Japanese Patent Kokoku No. Hei 4-15221) or as a remedy for diabetic complication and neuropathy (WO 0168136) were reported. It was also reported that a combination of nateglinide with another medicine is usable for the prevention, inhibition of advance and treatment of the following diseases and conditions associated with abnormal metabolism, particularly diabetes mellitus: hyperglycemia, hyperinsulinemia, hyperlipemia, insulin resistance, impaired glucose metabolism, obesity, diabetic retinopathy, macular degeneration, cataracts, diabetic nephropathy, glomerulosclerosis, diabetic neuropathy, erectile dysfunction, premenstrual syndrome, vascular restenosis, ulcerative colitis, coronary heart disease, hypertension, angina, myocardial infarction, stroke, disorders of skin and connective tissue, foot ulcerations, metabolic acidosis, arthritis, osteoporosis and impaired glucose tolerance (WO 01/21159, WO 01/26639). However, the effects of nateglinide on the expression of ACL have not been reported. It was also reported that insulin stimulates the expression of ACL to the contrary (Yonsei. Med. J., 35: 25 (1994)). Thus, there has never been reported that nateglinide suppresses the expression of ACL specific to the metabolic syndrome and that nateglinide is effective on hepatic diseases related to metabolic syndrome, particularly abnormal lipid metabolism.

On the other hand, although many cases of clinical results with insulin secretagogue have been reported, the effects thereof on liver diseases and liver functions are yet unknown. For example, it was reported that no significant alterations in liver function were observed after 30 days' administration of nateglinide (Drugs R&D; 2(2): 123 (1999)). However, in this experiment, the patients did not suffer from liver diseases. It was reported that hepatic cirrhosis had no significant effect on the pharmacokinetics of nateglinide (J. Clin. Pharmacol., 40: 634 (2000), Drugs, 60(3): 607 (2000)) and that nateglinide must be carefully given to patients with chronic liver diseases because the total exposure was increased by 30 % in subjects with mild hepatic impairment (Am J Health-Syst Pharm, 58, 285 (2001)). However, these reports are not for describing the effects of the medicine on the liver diseases. Incremental liver function test values (GOT, GPT), that were considered to be possibly related to the study drug, have been reported infrequently (Diabetes Care 23; 202 (2000), Diabetes Care 24; 73 (2001), Ann. Pharmacother. 35: 1426 (2001)). However, also these reports only relate to the safety of the medicines and the effects of the medicines on liver diseases are not described therein. It was also reported that hepatic enzyme levels is increased with the combination of nateglinide with thiazolidinedion (troglitazone) (Am J Health-Syst Pharm, 58 1200 (2001), Diabetes Care 25; 1529 (2002)). However, it is described therein that this increase is due to the treatment with troglitazone. Namely, it has never been reported at all that an insulin secretagogue such as nateglinide improves liver diseases or liver function (hepatic enzyme levels such as GOT or GPT).

### Disclosure of the Invention

An object of the present invention is to provide a composition for preventing, improving and treating NASH.

Another object of the present invention is to provide a composition for preventing, improving and treating other liver disorders.

As described above, the development of a medicine having a new action mechanism and effective in preventing, improving and treating metabolic syndrome, particularly liver diseases with abnormal lipid metabolism is demanded.

After intensive investigations made for the purpose of solving the above-described problems, the inventors have found that the elevation of the expression of liver ACL specific for the metabolic syndrome is recognized on the gene expression level in Goto-Kakizaki rats (hereinafter referred to as "GK rats") which suffer from hyperglycemia caused by insufficient insulin secretion in response to glucose and that surprisingly, when an insulin secretagogue, particularly that having an rapid effect such as a meglitinide, e. g. nateglinide, is administered, elevated liver ACL expression of GK rats can be suppressed. The present invention has been completed on the basis of this finding.

Diabetes is considered to be one of phenotypes of metabolic syndrome. It was confirmed that when nateglinide was administered for 3 months to patients suffering from diabetes and having fatty liver or mild liver failures, GOT and GPT which are indices of the liver functions of these patients were improved.

Namely, the present invention provides a pharmaceutical composition comprising a meglitinide, for use in preventing, alleviating or treating NASH or another liver disorder, the meglitinide being selected from:
nateglinide, of formula: 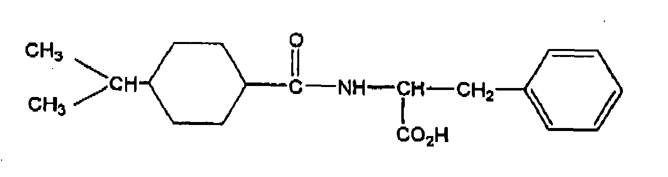
KAD-1229, of formula: 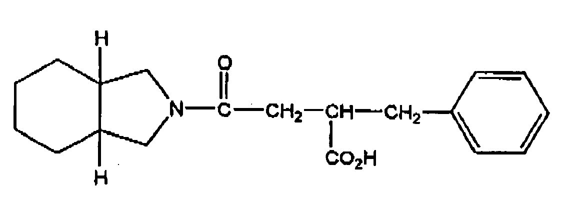
repaglinide of formula: 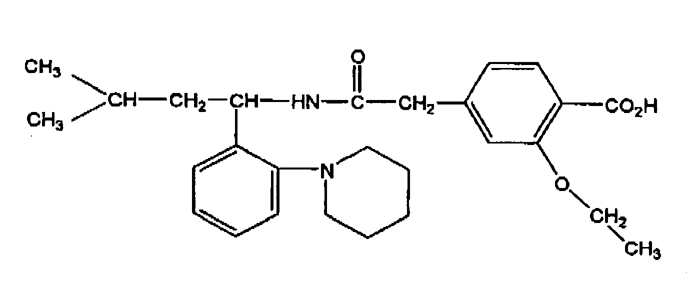 and compounds of formula (I): 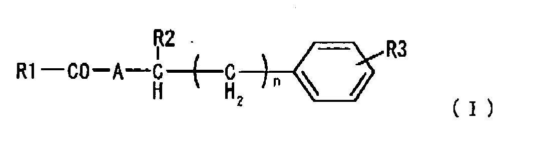 wherein R1 represents a group represented by the following formula: 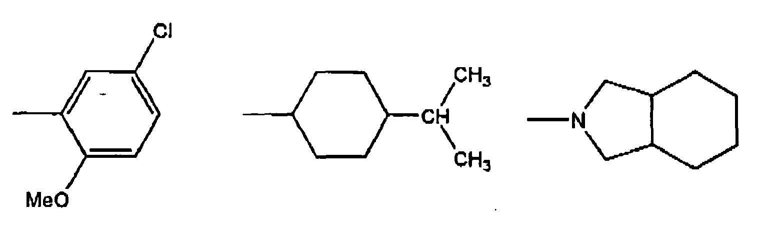 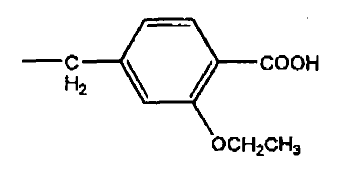 R2 represents a hydrogen atom, a methyl group or ethyl group, R3 represents hydrogen atom, carboxyl group or 1-piperidyl group, A represents NH or CH₂ group, and n represents 0 or 1.

The present invention also provides use of the above-described compound for producing a composition for preventing, improving and treating NASH or other liver disorder.

The compounds used in the present invention for suppression of the expression of ACL include those of the following general formula (I) (Hormone and Metabolic Research Journal , Vol. 27, 263-266 (1995)) 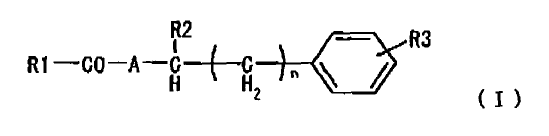 wherein R1 represents a group represented by the following formula: 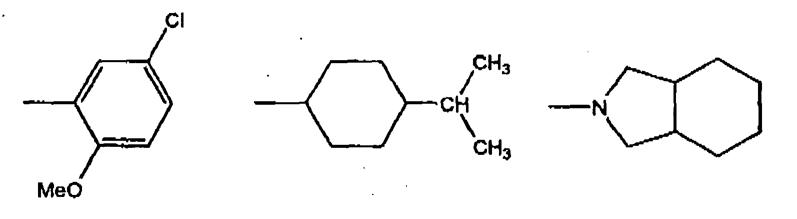 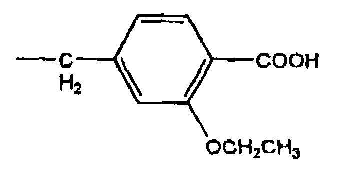 R2 represents hydrogen atom, a lower alkyl group such as methyl group or ethyl group, R3 represents hydrogen atom, carboxyl group or 1-piperidyl group, A represents NH or CH₂ group, and n represents 0 or 1.

More preferred compounds are D-phenylalanine derivatives such as (-)-N-(trans-4-isopropylcyclohexanecarbonyl)-D-phenylalanine (hereinafter referred to as "nateglinide"), benzylsuccinic acid derivatives such as mitiglinide and benzoic acid derivatives such as repaglinide. In them, the most preferred compound is nateglinide:

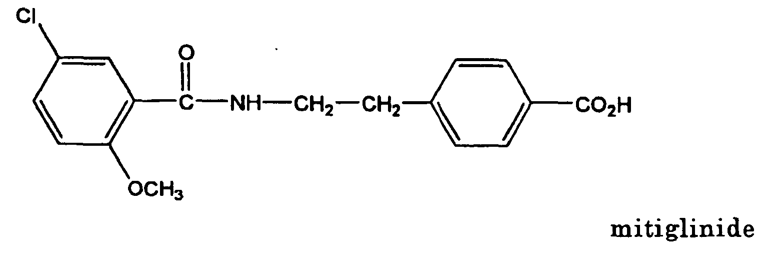

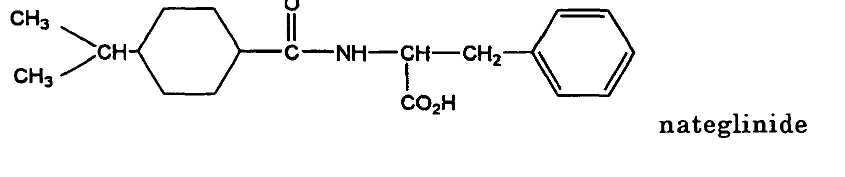

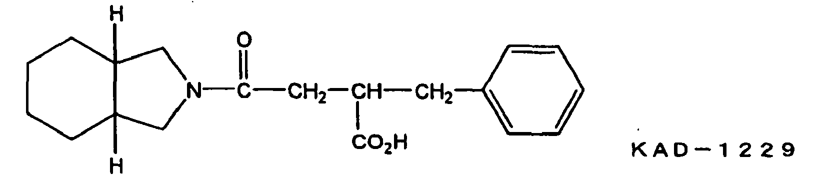

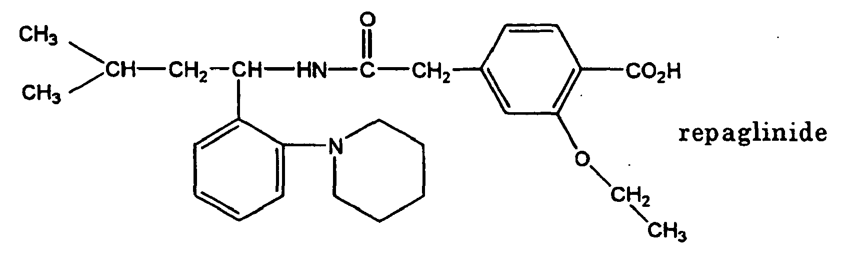

In the present invention, the above-described compounds are usable either alone or in combination with at least one of hypoglycemic agents and therapeutic agent for hyperlipemia. The hypoglycemic agents include, for example, insulin and insulin derivatives such as lispro and glargine, sulfonylureas such as tolbutamide, gliclazide, glibenclamide and glimepiride, α glucosidase inhibitors such as acarbose, voglibose and miglitol, insulin sensitizers such as biguanides, e. g. metformin and phenformin, thiazolidines, e. g. pioglitazone, rosiglitazone and troglitazone and PPAR γ agonists and antagonists having non-thiazolidine structures such as GI-262570, JTT-501, YM-440, NN-622 and KRP-297, adrenaline β3 receptor agonists such as AJ-9677, insulin-like agonists such as CLX-0901, GLP-1 agonists such as GLP-1, Exendin-4 and NN-2211, DPPIV inhibitors such as DPP-728A, SGLT inhibitors such as T-1095 and ACC inhibitors.

The therapeutic agents for hyperlipemia, which lowers blood lipids, include HMG-CoA reductase inhibitors such as pravastatin, simvastatin, fluvastatine, cerivastatin, atorvastatin and itavastatin, fibrate such as simfibrate, clofibrate, clinofibrate, bezafibrate and fenofibrate, anion exchange resins such as colestimide and colestyramine, and nicotinic acid preparations such as nicomol and niceritrol. By using a combination of them, the effect of the present invention can be further improved. Such a combination is very effective on metabolic syndrome, in particular, abnormal lipid metabolism. The term "combination" means either that two of them are contained in a pharmaceutical composition or that two pharmaceutical compositions containing a different ingredient are administered to the patients at the same time or at an interval.

Although the dose of the compound which suppresses the expression of ACL in the pharmaceutical composition, hypoglycemic agent and/or therapeutic agent for hyperlipemia is not particularly limited, the content of the compound which suppresses the expression of ACL in the composition is preferably 0.1 to 99 % by weight and that of the hypoglycemic agent and/or therapeutic agent for hyperlipemia is preferably 1 to 99.9 % by weight.

The pharmaceutical composition of the present invention can contain, in addition to the above-mentioned ingredients, pharmacologically allowable various substances (as adjuvants) for the preparation (hereinafter referred to as pharmaceutically allowable carriers). The materials for the preparation can be suitably selected depending on the dosage form of the preparation. They include, for example, excipients, diluents, additives, disintegrators, binders, coating agents, lubricants, gliding agent, glazing agents, flavors, sweetening agents and solubilizers. Examples of the materials for the preparation include magnesium carbonate, titanium dioxide, lactose, mannitol and other saccharides, talc, milk protein, gelatin, starch, cellulose and derivatives thereof, animal oils, vegetable oils, polyethylene glycol and solvents such as sterilized water and monohydric or polyhydric alcohols, e. g. glycerol.

The pharmaceutical composition of the present invention is effective for preventing, improving and treating various liver disorders, including NASH.

The pharmaceutical preparation of the present invention can be prepared suitably for various administration methods such as oral administration, intraperitoneal administration, percutaneous administration and inhalation administration. Concretely, the pharmaceutical preparation can be in a suitable solid or liquid form such as granules, powder, coated tablets, tablets, (micro)capsules, suppositories, syrup, juice, suspension, emulsion, dropping agent, solution for injection and a preparation capable of elongating the release of the active agent.

The dose of the compound (active ingredient) which suppresses the expression of ACL used for the medicine of the present invention is suitably determined depending on the variety of the compound which suppresses the expression of ACL, kind of complication, extent of the symptoms of the complication and neuropathy, dosage form and side effects of the preparation and degree thereof. For example, when a preparation containing nateglinide as the active ingredient is orally administered, the dose thereof is preferably about 10 mg to 10 g, more preferably about 30 mg to 1 g and most preferably about 90 to 270 mg in terms of the net weight of nateglinide. The dose can be further increased when the patient is under serious conditions. As for the number of times and timing of the medication, the medicine can be administered once / several days or once a day. Usually, the medicine is administered several times a day, for example, 2 to 4 times a day, preferably before meal. In the parenteral administration such as intravenous administration, the dose may be about 1/10 to 1/20 of the dose in the oral administration.

When one of hypoglycemic agents and therapeutic agents for hyperlipemia is used or when two or more of them are used in the form of a mixture or in combination, the dose or the amount of each of them can be the same as that of the hypoglycemic agent and/or therapeutic agent for hyperlipemia or each of the medicines already developed or being developed as medicines having the intended effects.

The following Examples will further illustrate the present invention.

### Example 1

The effect of nateglinide on the expression of liver ACL was analyzed with DNA chips.

In Experiment 1, methylcellulose or 50 mg/kg of nateglinide suspended in methylcellulose was orally administered to normal Wistar rats fasted overnight. One hour after the administration, the liver was taken from each rat and freezed. The total RNA was extracted with RNeasy kit (Qiagen Co. Ltd). Biotinylated cRNA probe was prepared by a standard method and then it was hybridized to rat genome U34 array (Affymetrix Co. Ltd). The amount of cRNA hybridized to rat ACL gene (GenBank ID: J05210) was analyzed with Microarray Suite 5.0 software (Affymetrix Co. Ltd).

Also in Experiment 2, 1 g/kg of glucose, 50 mg/kg of nateglinide or both of glucose and nateglinide were orally administered to Wistar rats and GK rats fasted overnight. One hour after the administration, the liver was taken from each rat and the expression of ACL level was analyzed with DNA chips in the same manner as that in Experiment 1.

**Table 1**

| (Experiment 1) | | | | | |
|---|---|---|---|---|---|
| Group | Rat | Glucose | Nateglinide | Analysis 1 | Analysis 2 |
| 1 | Wistar | - | - | 96.2 | 459.3 |
| 2 | Wistar | - | + | 144.4 | 543.6 |

| (Experiment 2) | | | | | |
|---|---|---|---|---|---|
| Group | Rat | Glucose | Nateglinide | Analysis 1 | Analysis 2 |
| 1 | Wistar | - | - | 79.2 | 257.3 |
| 2 | Wistar | + | - | 63.5 | 282 |
| 3 | GK | - | - | 139.6 | 452.5 |
| 4 | GK rat | + | - | 139.6 | 416.7 |
| 5 | GK rat | + | + | 66.4 | 189.9 |

The expression level of liver ACL was constitutively elevated in GK rats as compared with normal Wistar rats (Experiment 2, lvs3).

It is considered that the load of glucose on normal rats increases the blood glucose level and also causes normal portal insulin secretion. However, even when normal rats were exposed to high glucose and high insulin, the expression level of liver ACL genes was not suppressed one hour after the administration (Experiment 2, lvs2). Also in GK rats, no significant change was found in the expression of liver ACL by the load with glucose (Experiment 2, 3vs4). When nateglinide was administered to normal Wistar rats, no significant suppression of liver ACL mRNA level was recognized (Experiment 1).

On the contrary, when nateglinide was administered to GK rats (Experiment 2, 3,4vs5), surprisingly, the elevated ACL expression level was significantly suppressed and returned to the normal level. By the administration of nateglinide, transient increase in insulin level in GK rat portal vein was observed.

Thus, it was confirmed that by the administration of nateglinide, the elevated liver ACL expression level of GK rats was remarkably improved.

### Example 2

Nateglinide was orally administered to patients with type 2 diabetes three times a day in a dose of usually 90 mg/day before meals for 12 weeks. Blood GOT and GPT concentrations were determined as indices of the liver function in 0 week and 12^{th} week.

In analysis 1, the results obtained by the oral administration of nateglinide to 53 patients with type 2 diabetes each having fatty liver for 12 weeks were analyzed. The results are shown in Table 2.

**Table 2**

| | 0 week | 12^{th} week | (0 to 12 weeks) | p |
|---|---|---|---|---|
| GOT (IU/L) | 58.4±34.4 | 33.8±22.9 | 24.7±12.4 | <0.05 |
| GPT (IU/L) | 77.2±55.9 | 44.0±29.7 | 33.2±31.0 | <0.05 |

Levels of GOT and GPT after the administration of nateglinide statistically significant lowered , compared with those before the administration thereof.

In 5 cases with fatty liver and having NASH-like liver troubles (GOT, GPT ≧ 51 IU/L, GPT>GOT,), GOT value was reduced from 138.6±56.0 to 83.6±41.5, and GPT value was reduced from 242.6±112.8 to 57.8±59.2.

In analysis 2, the results obtained by the oral administration of nateglinide to 16 cases of type-2 diabetes having liver disorders (GOT, GPT ≧ 51 IU/L) were analyzed. The results are shown in Table 3.

**Table 3**

| | | | | |
|---|---|---|---|---|
| | 0 week | 12^{th} week | 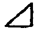 (0 to 12 weeks) | p |
| GOT (IU/L) | 133.3±51.3 | 85.4±40.9 | 47.8±37.9 | <0.05 |
| GPT (IU/L) | 217.3±94.1 | 80.2±53.4 | 137.0±43.4 | <0.05 |

In the comparison of GOT and GPT before the administration of nateglinide with those after the administration thereof, a statistically significant lowering in both GOT and GPT was recognized.

It was thus suggested that the liver function of patients with fatty liver, NASH or liver disorders can be improved by the administration of nateglinide.

Thus, nateglinide can be expected as a medicine for the prevention, improvement or treatment of liver disorders caused by metabolic syndrome, particularly abnormal lipid metabolism.

The present invention can provide medicines for the prevention, improvement, treatment, etc. of metabolic syndrome, fatty liver, NASH and liver disorders, methods for using the medicines (e. g. method of the administration for the treatment of these diseases) and use of the compounds, capable of suppression of ACL expression for this purpose, for the preparation of the medicine. In particular, nateglinide is expected as the compound for suppression of the expression of ACL.

## Claims

1. A pharmaceutical composition comprising a meglitinide, for use in preventing, alleviating or treating NASH or another liver disorder, the meglitinide being selected from:
nateglinide, of formula: 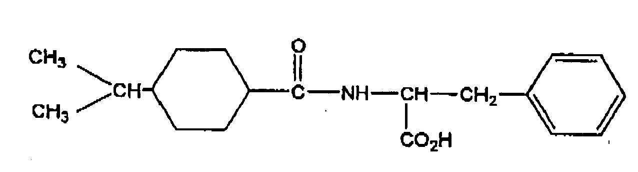 KAD-1229, of formula: 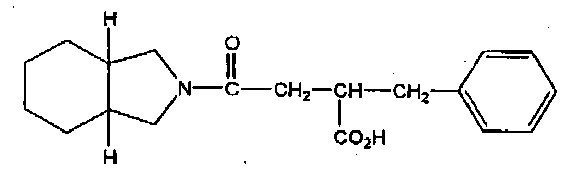
repaglinide of formula: 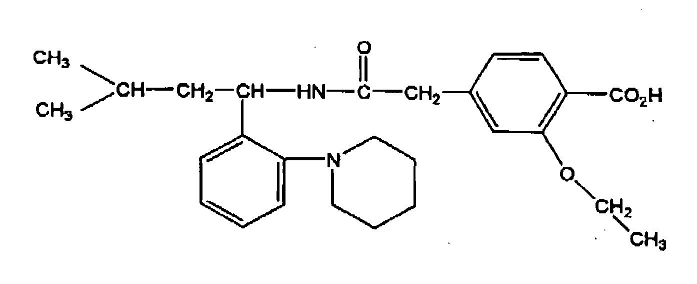 and compounds of formula (I): 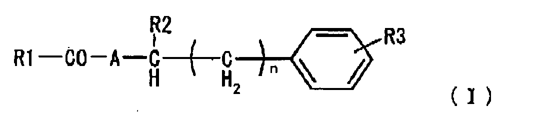 wherein R1 represents a group represented by the following formula: 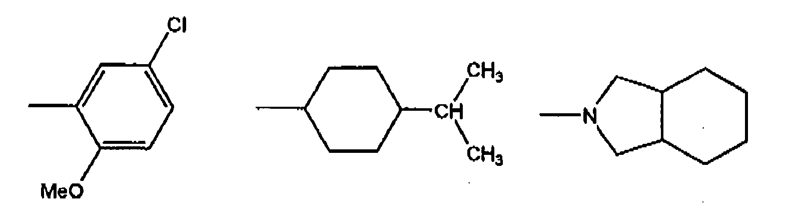 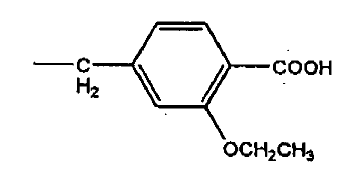 R2 represents a hydrogen atom, a methyl group or ethyl group, R3 represents hydrogen atom, carboxyl group or 1-piperidyl group, A represents NH or CH₂ group,
and n represents 0 or 1.

2. A pharmaceutical composition according to claim 1 for use in preventing, alleviating or treating NASH or another liver disorder, wherein the meglitinide is nateglinide.

3. A pharmaceutical composition according to claim 1 or claim 2 for use in preventing, alleviating or treating NASH.

4. A pharmaceutical composition according to any preceding claim for use in preventing, alleviating or treating NASH or another liver disorder, wherein said composition further contains at least one of hypoglycaemic agents or therapeutic agents for hyperlipemia.

5. Use of a meglitinide selected from:
nateglinide, of formula: 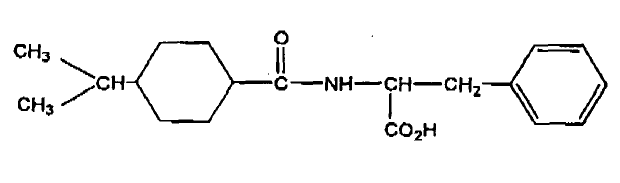 KAD-1229, of formula: 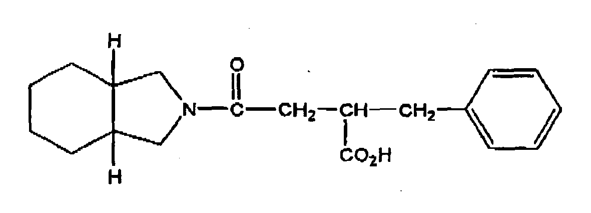
repaglinide of formula: 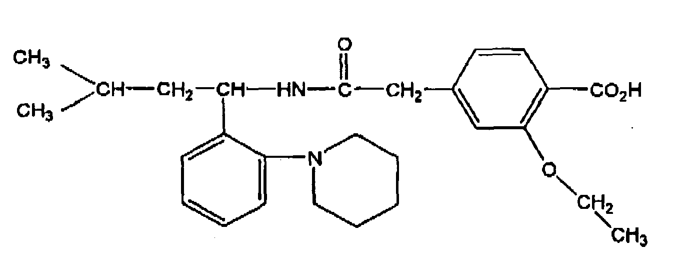 and compounds of formula (I): 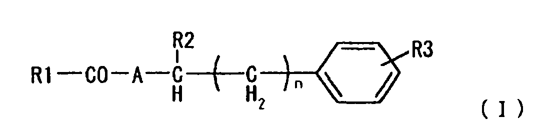 wherein R1 represents a group represented by the following formula: 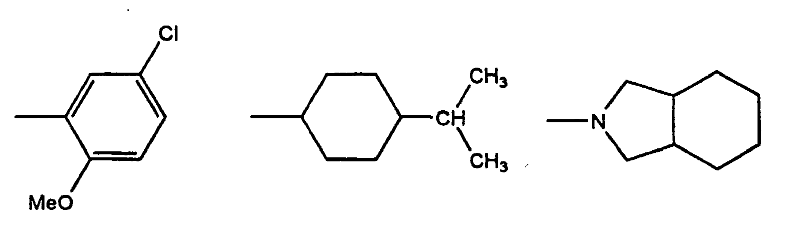 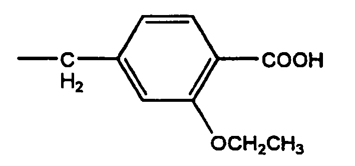 R2 represents a hydrogen atom, a methyl group or ethyl group, R3 represents hydrogen atom, carboxyl group or 1-piperidyl group, A represents NH or CH₂ group, and n represents 0 or 1, in the manufacture of a composition for use in preventing alleviating or treating NASH or another liver disorder.

6. A use according to claim 5 wherein the meglitinide is nateglinide.

7. A use according to claim 5 or claim 6 wherein the composition is for use in preventing, alleviating or treating NASH.

8. A use according to any one of claims 5 to 7, wherein the composition further contains at least one of hypoglycaemic agents or therapeutic agents for hyperlipidemia.

## Patentansprüche

1. Pharmazeutische Zusammensetzung, umfassend ein Meglitinid, zur Verwendung beim Verhindern, Lindern oder Behandeln von NASH oder einer anderen Lebererkrankung, wobei das Meglitinid ausgewählt ist aus:
Nateglinid mit der Formel: 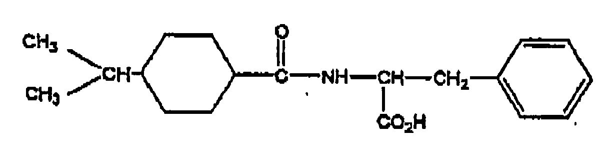
KAD-1229 mit der Formel: 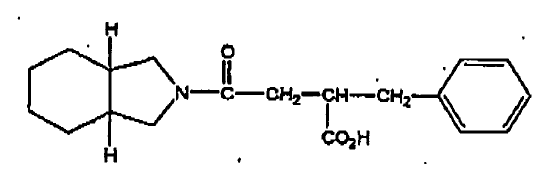
Repaglinid mit der Formel: 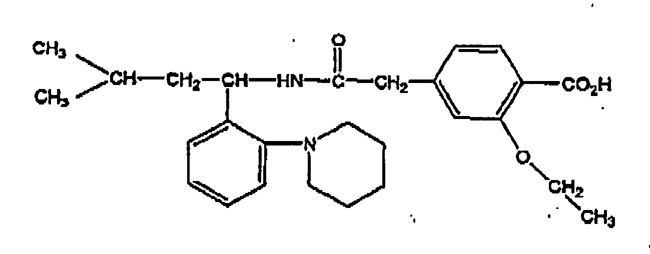 und Verbindungen der Formel (I): 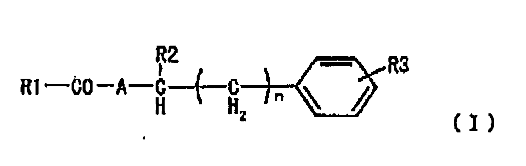 worin R1 eine Gruppe repräsentiert, die durch eine im Folgenden aufgeführte Formel dargestellt ist: 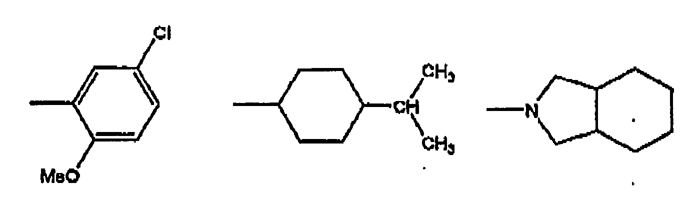 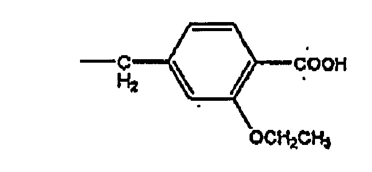 R2 ein Wasserstoffatom, eine Methylgruppe oder Ethylgruppe darstellt, R3 ein Wasserstoffatom, eine Carboxylgruppe oder 1-Piperidyl-Gruppe darstellt, A NH oder
eine CH₂-Gruppe darstellt, und n 0 oder 1 darstellt.

2. Pharmazeutische Zusammensetzung nach Anspruch 1 zur Verwendung beim Verhindern, Lindern oder Behandeln von NASH oder einer anderen Lebererkrankung, wobei das Meglitinid Nateglinid ist.

3. Pharmazeutische Zusammensetzung nach Anspruch 1 oder 2 zur Verwendung beim Verhindern, Lindern oder Behandeln von NASH.

4. Pharmazeutische Zusammensetzung nach einem der vorstehenden Ansprüche zur Verwendung beim Verhindern, Lindern oder Behandeln von NASH oder einer anderen Lebererkrankung, wobei die Zusammensetzung weiterhin wenigstens eines von hypoglykämischen Mitteln oder therapeutischen Mitteln für Hyperlipidämie enthält.

5. Verwendung eines Meglitinids, ausgewählt aus:
Nateglinid mit der Formel: 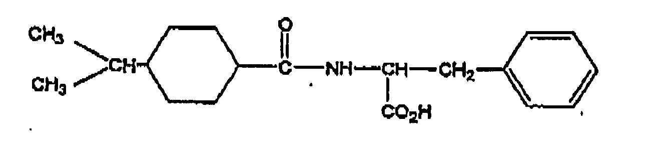 KAD-1229 mit der Formel: 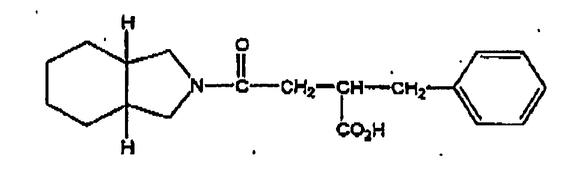
Repaglinid mit der Formel: 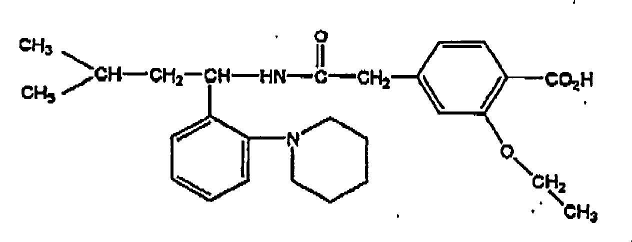 und Verbindungen der Formel (I): 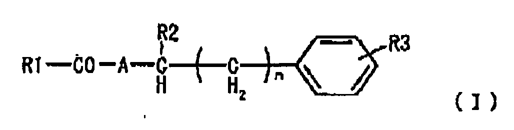 worin R1 eine Gruppe repräsentiert, die durch eine im Folgenden aufgeführte Formel dargestellt ist: 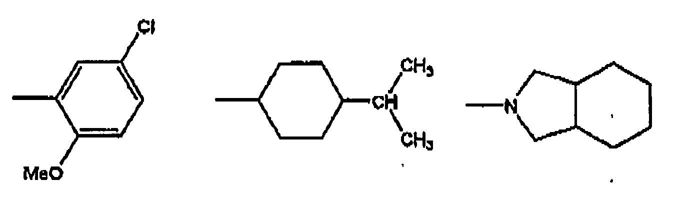 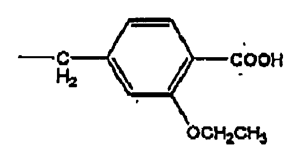 R2 ein Wasserstoffatom, eine Methylgruppe oder Ethylgruppe darstellt, R3 ein Wasserstoffatom, eine Carboxylgruppe oder 1-Piperidyl-Gruppe darstellt, A NH oder eine CH₂-Gruppe darstellt, und n 0 oder 1 darstellt, bei der Herstellung einer Zusammensetzung zur Verwendung beim Verhindern, Lindern oder Behandeln von NASH oder einer anderen Lebererkrankung.

6. Verwendung nach Anspruch 5, wobei das Meglitinid Nateglinid ist

7. Verwendung nach Anspruch 5 oder 6, wobei die Zusammensetzung zur Verwendung beim Verhindern, Lindern oder Behandeln von NASH vorgesehen ist.

8. Verwendung nach einem der Ansprüche 5 bis 7, wobei die Zusammensetzung weiterhin wenigstens eines von hypoglykämischen Mitteln oder therapeutischen Mitteln für Hyperlipidämie enthält.

## Revendications

1. Composition pharmaceutique comprenant un méglitinide, destinée à être utilisée pour prévenir, soulager ou traiter la stéatohépatite non alcoolique ou un autre trouble du foie, le méglitinide étant choisi parmi :
le natéglinide de formule: 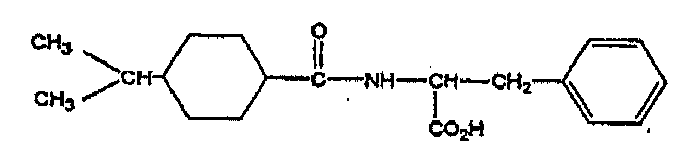
le KAD-1229 de formule : 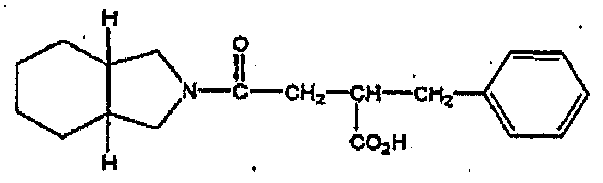
le répaglinide de formule : 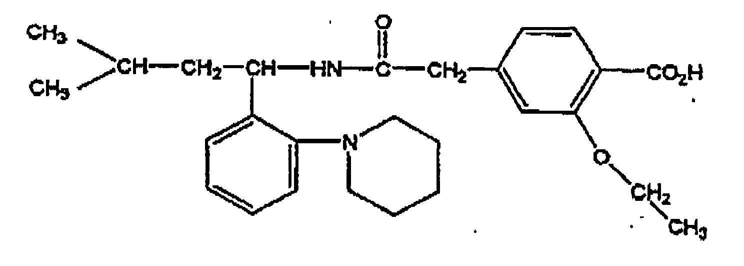 et les composés de formule (I): 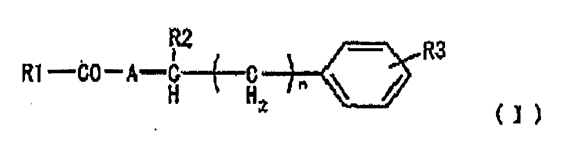 dans laquelle R1 représente un groupe représenté par la formule suivante : 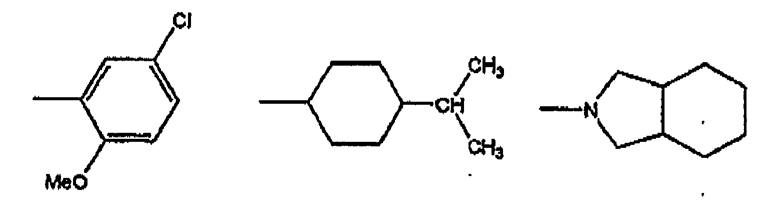 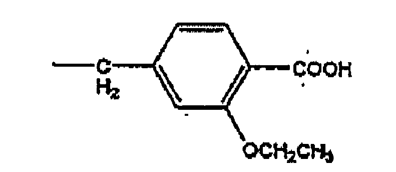 R2 représente un atome d'hydrogène, un groupe méthyle ou groupe éthyle, R3 représente un atome d'hydrogène, un groupe carboxyle ou un groupe 1-pipéridyle, A représente un groupe NH ou CH₂ et n représente 0 ou 1.

2. Composition pharmaceutique selon la revendication 1 destinée à être utilisée pour prévenir, soulager ou traiter la stéatohépatite non alcoolique ou un autre trouble du foie, dans laquelle le méglitinide est un natéglinide.

3. Composition pharmaceutique selon la revendication 1 ou la revendication 2, destinée à être utilisée pour prévenir, soulager ou traiter la stéatohépatite non alcoolique.

4. Composition pharmaceutique selon l'une quelconque des revendications précédentes destinée à être utilisée pour prévenir, soulager ou traiter la stéatohépatite non alcoolique ou un autre trouble du foie, dans laquelle ladite composition contient en outre au moins un des agents hypoglycémiques ou agents thérapeutiques pour l'hyperlipémie.

5. Utilisation d'un méglitinide choisi parmi :
le natéglinide de formule: 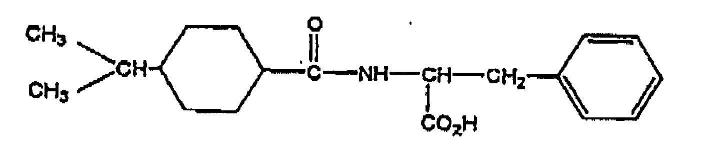
le KAD-1229 de formule : 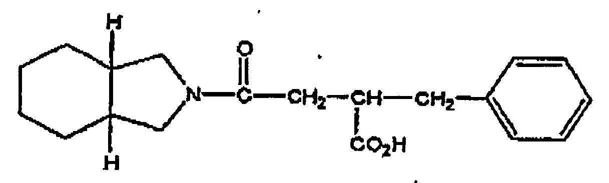
le répaglinide de formule : 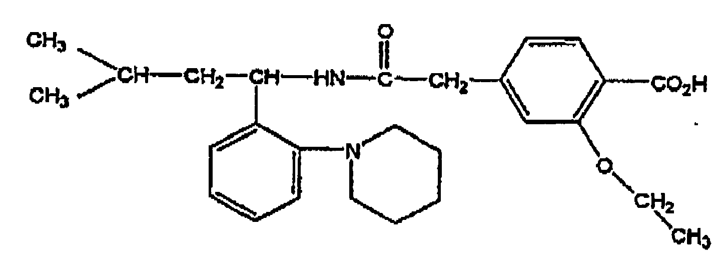 et les composés de formule (I): 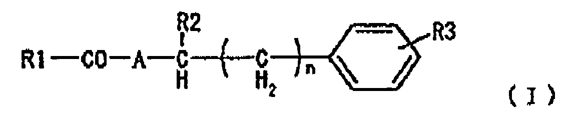 dans laquelle R1 représente un groupe représenté par la formule suivante : 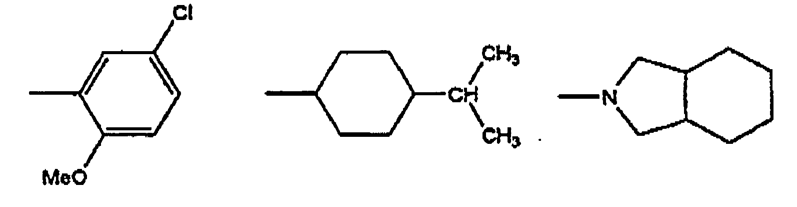 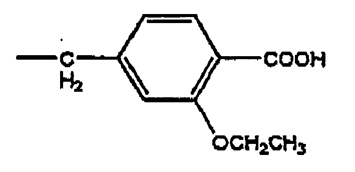 R2 représente un atome d'hydrogène, un groupe méthyle ou groupe éthyle, R3 représente un atome d'hydrogène, un groupe carboxyle ou un groupe 1-pipéridyle, A représente un groupe NH ou CH₂ et n représente 0 ou 1, dans la préparation d'une composition destinée à être utilisée pour prévenir, soulager ou traiter la stéatohépatite non alcoolique ou un autre trouble du foie.

6. Utilisation selon la revendication 5, dans laquelle le méglitinide est un natéglinide.

7. Utilisation selon la revendication 5 ou la revendication 6, dans laquelle la composition sert à prévenir, soulager ou traiter la stéatohépatite non alcoolique.

8. Utilisation selon l'une quelconque des revendications 5 à 7, dans laquelle la composition contient en outre au moins un des agents hypoglycémiques ou agents thérapeutiques pour l'hyperlipidémie.
